# EUROPEAN PATENT APPLICATION

(11) **EP 3 838 120 A1**
(43) Date of publication of application: **23.06.2021**
(21) Application number: 20151812.3
(22) Date of filing: 14.01.2020
(51) Int. Cl.: A61B 5/00

(54) **PHARMACOLOGICALLY ANNOTATION BASED SLEEP QUALITY DETECTION**

(30) Priority: 20.12.2019 US 201962951533 P
(71) Applicant: Koninklijke Philips N.V., 5656 AG Eindhoven (NL)
(72) Inventor: GHOSH, Erina, 5656 AE Eindhoven (NL); ALTIMIER, Leslie, 5656 AE Eindhoven (NL)
(74) Representative: Steenbeek, Leonardus Johannes

(57) **Abstract**

A sleep quality detection device (50) employs a waveform pre-processor (60) and a sleep quality classifier (70). In operation, the waveform pre-processor (60) derives a plurality of monitoring waveform segments from a temporal segmentation of one or more monitoring waveforms annotated with pharmacological data (e.g., an annotation of physiological waveform(s) and/or psychological waveforms with a temporal administration of sedative drug(s)). The sleep quality classifier (70) includes one or more machine learning models (e.g., artificial neural network(s) and/or deep learning network(s)) trained to individually classify one or more of the monitoring waveform segments as either a natural sleep waveform segment or a drug-induced sleep waveform segment. Alternatively, the machine learning model(s) may be trained to individually classify each monitoring waveform segment as either a natural sleep waveform segment, a drug-induced sleep waveform segment or a conscious waveform segment.

## Description

### FIELD OF THE INVENTION

The present disclosure generally relates to evaluating sleep quality and changes in sleep patterns over time. The present disclosure specifically relates to identifying a patient sleep state from pharmacologically annotated physiological waveforms (e.g., EEG or ECG) and/or psychological waveforms and classifying the identified patient sleep state as drug-induced sleep or natural sleep.

### BACKGROUND OF THE INVENTION

Sleep is regulated by body clock and is characterized by reduced consciousness and inhibition of voluntary muscle activity/sensory activity. Sleep is critically important for restoring the immune, nervous, skeletal and muscular systems and helps improve cognitive function, memory and mood.

Sleep consists of a rapid eye movement (REM) stage and non- rapid eye movement (NREM) stage. A person cycles between REM sleep and NREM sleep multiple times during a night's sleep.

People who have difficulty sleeping are often prescribed sedatives, which are central nervous system depressants for inducing sleep by slowing brain activity. However, although sedatives help with relaxation, the sleep patterns and quality of sleep using sedatives is different from natural sleep.

Quantifying sleep patterns in patients provides valuable information on a patient's state. For example, restoration of normal sleep patterns from drug-induced sleep patterns indicate that the patient is recovering well and can be prescribed lower doses of sedatives or no sedatives. Conversely, adverse changes in sleep patterns, longer periods of wakefulness and restlessness could indicate deterioration or poorly managed pain. Therefore, quantifying sleep patterns taking into account sedatives provides useful information.

Further, quality of sleep is an important factor of recovery from illness. It is also indicator of comfort and pain levels. However, current measurements of sleep quality are subjective (self-reported) or require sleep studies in special sleep research centers, which is not a viable solution for continuous, unobtrusive monitoring. Moreover, current measurements of sleep quality don't include information about patient's health status and the medications they are taking.

### SUMMARY OF THE INVENTION

It is an object of the invention to provide a pharmacologically annotation based sleep quality detection. The invention is defined by the independent claims. The dependent claims define advantageous embodiments.

The present disclosure describes a categorical classification or a quantitative classification of a sleeping status of a subject as a natural sleep or a drug-induced sleep based on physiological and/or psychological monitoring/reporting of the subject annotated with any relevant pharmacological status of the subject.

The present disclosure also describes a categorical classification or a quantitative classification of an awareness status of the subject as a conscious state or a sleep state based on physiological and/or psychological monitoring/reporting of the subject.

The present disclosure may be embodied as:
(1) a sleep quality detection device of the present disclosure;
(2) a sleep quality detection controller of the present disclosure;
(3) a sleep quality detection system of the present disclosure; and
(4) a sleep quality detection method utilizing a sleep quality detection device of the present disclosure.

Various sleep quality detection device embodiments of the present disclosure encompass a waveform data processor and a sleep quality classifier for detecting a quality of sleep.

In operation, the waveform data processor drives a plurality of monitoring waveform segments from a temporal segmentation of one or more monitoring waveforms (e.g., physiological waveform(s) and/or psychological waveform(s)) temporally annotated with pharmacological data.

Examples of a physiological waveform include, but are not limited to, electrocardiograms (ECG), electroencephalogram (EEG), a photoplethysmogram (PPG) and a capnogram (CO₂).

Examples of a psychological waveform include, but are not limited to, chest well waveform, a thorax waveform and an abdomen waveform.

Pharmacological data is informative of a temporal administration of sedative drugs to a subject being monitored for sedated sleep quality.

The sleep quality classifier includes one or more machine learning models (e.g., supervised learning model(s) or semi-supervised learning model(s)) trained to individually classify one or more of the monitoring waveform segments as a natural sleep waveform segment or a drug-induced sleep waveform segment.

The machine learning model(s) may be further trained to individually classify each monitoring waveform segment as one of a natural sleep waveform segment, a drug-induced sleep waveform segment or a conscious waveform segment.

The machine learning model(s) may implement one or more classification techniques as known in the art of the present disclosure or herein after conceived for categorially classifying a monitoring waveform segment as a natural sleep waveform segment or a drug-induced sleep waveform segment (or a conscious waveform segment if applicable). Examples of a classification technique include, but are not limited to, an artificial neural network, a deep learning neural network, a support vector machine (SVM), a decision tree, a random forest, a *k-*nearest neighbor, a Naive Bayes, a discriminant analysis and a logistic regression.

The machine learning model(s) may implement one or more regression techniques as known in the art of the present disclosure or herein after conceived for quantifiably classifying a monitoring waveform segment as a natural sleep waveform segment or a drug-induced sleep waveform segment (or a conscious waveform segment if applicable). Examples of a regression technique include, but are not limited to, an artificial neural network, a deep learning neural network, a decision tree, a linear model, a non-linear model, a regularization, a stepwise regression and an adaptive neuro-fuzzing learning.

Various sleep quality detection controller embodiments of the present disclosure encompass one or more processors and a non-transitory machine-readable storage medium encoded with instructions executable by the processor(s) to derive a plurality of monitoring waveform segments from a temporal segmentation of one or more monitoring waveforms (e.g., physiological waveform(s) and/or psychological waveform(s)) temporally annotated with pharmacological data, and further to individually classify, via one or more machine learning models (e.g., supervised learning model(s) or semi-supervised learning model(s)), one or more of the monitoring waveform segments as a natural sleep waveform segment or a drug-induced sleep waveform segment.

The instructions may further individually classify each monitoring waveform segment as one of a natural sleep waveform segment, a drug-induced sleep waveform segment or a conscious waveform segment.

Various sleep quality detection system embodiments of the present disclosure encompass a sleep quality detection device(controller) of the present disclosure and further encompass one or more physiological waveform sources, one or more psychological waveform source(s), and one or more pharmacological sources.

Examples of a physiological waveform source include, but are not limited, a heart rate monitor, a ECG monitor, a blood pressure monitor, a pulse oxygen saturation monitor, a respiratory motion sensor, a patient physiological input file generated by a medical sedation system and a patient physiological record maintained by an electronic medical record system.

Examples of a psychological waveform source include, but are not limited, a camera for monitoring a body movement of patient, a motion sensor for monitoring a body movement of patient, a patient psychological input file generated by a medical sedation system and a patient psychological record maintained by an electronic medical record system.

Examples of a pharmacological data source include, but are not limited, operator manual input of patient pharmacologic information into a monitor, a patient pharmacologic input file generated by a medical sedation system and a patient pharmacologic record maintained by an electronic medical record system.

Various sleep quality detection method embodiments of the present disclosure encompass an operation of a sleep quality detection device(controller) of the present disclosure involving (1) deriving, via a waveform data processor, a plurality of monitoring waveform segments from a temporal segmentation of at least one monitoring waveform temporally annotated with pharmacological data; and (2) individually classifying, via one or more machine learning model, one or more of the monitoring waveform segments as a natural sleep waveform segment or a drug-induced sleep waveform segment.

A sleep quality detection method of the present disclosure may further involve individually classifying, via the machine learning model(s), each monitoring waveform segments as a natural sleep waveform segment, a drug-induced sleep waveform segment or a conscious waveform segment.

The foregoing embodiments and other embodiments of the present disclosure as well as various structures and advantages of the present disclosure will become further apparent from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1 illustrates a first exemplary embodiment of a sleep quality detection device in accordance with the present disclosure;
FIG. 2 illustrates a flow chart representative of an exemplary embodiment of a sleep quality detection method in accordance with the present disclosure;
FIGS. 3A and 3B illustrates flow charts representative of an exemplary embodiment of a monitoring waveform pre-processing method in accordance with the present disclosure;
FIGS. 4A-4D illustrate exemplary embodiment of a sleep quality classifier in accordance with the present disclosure;
FIG. 5 illustrates a flow chart representative of an exemplary embodiment of a sleep quality classifier training method in accordance with the present disclosure;
FIG. 6 illustrates a flow chart representative of an exemplary embodiment of a sleep pattern analysis method in accordance with the present disclosure; and
FIGS. 7A and 7B illustrate exemplary embodiments of a sleep quality detection controller in accordance with the present disclosure incorporated within a sleep quality detection controller in accordance with the present disclosure.

### DETAILED DESCRIPTION OF EMBODIMENTS

The present disclosure improves upon by the prior art by providing sleep quality detection devices, sleep quality detection controllers, sleep quality detection systems and sleep quality detection methods for a categorical classification or a quantitative classification of a sleeping status of a subject as a natural sleep or a drug-induced sleep based on physiological and/or psychological monitoring/reporting of the subject annotated with any relevant pharmacological status of the subject.

The present disclosure also describes for a categorical classification or a quantitative classification of an awareness status of the subject as a conscious state or a sleep state based on physiological and/or psychological monitoring/reporting of the subject.

The present disclosure is applicable to numerous and various sleep monitoring/reporting of a subject for any medical/research purpose.

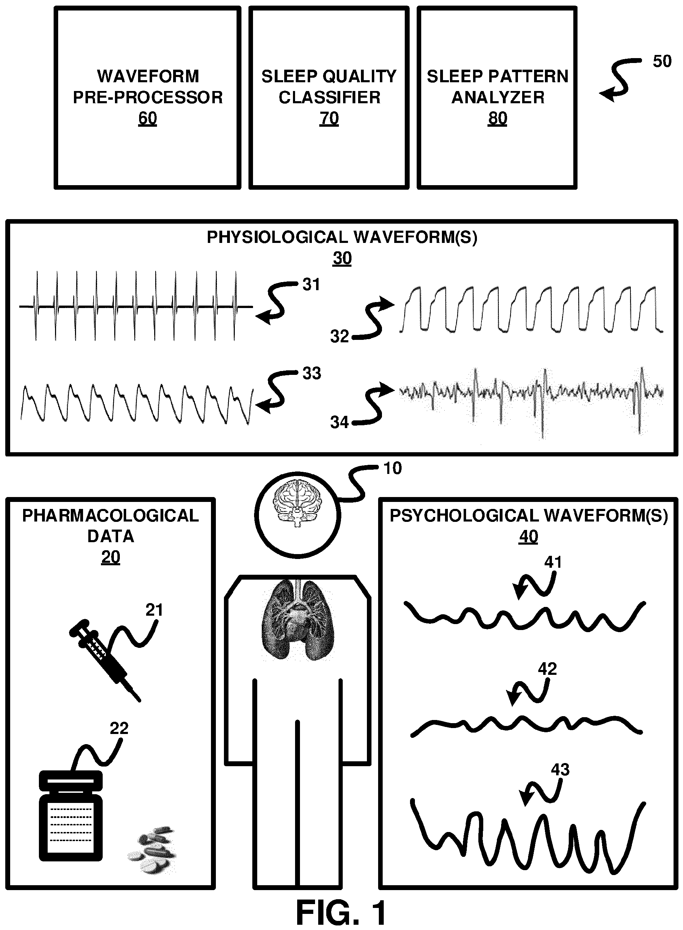

To facilitate an understanding of the present disclosure, the following description of FIGS. 1-6 teaches exemplary embodiments of sleep quality detection devices and sleep quality detection methods in accordance with the present disclosure. From the description of FIGS. 1-6, those having ordinary skill in the art of the present disclosure will appreciate how to apply the present disclosure to make and use additional embodiments of sleep quality detection devices and sleep quality detection methods in accordance with the present disclosure.

Referring to FIG. 1, a subject 10 is undergoing a sleep monitoring in a setting, clinical or non-clinical, involving an administration of sedatives via needle(s) 21 and/or pill(s) 22 to subject 10 for purposes of inducing sleep by slowing brain and cardiovascular activity of subject 10.

Pharmacological data 20 is informative of the sedative drugs given to subject 10 (e.g., name of drug, dose, time and mode of administration) to facilitate a monitoring of pharmacokinetical effect(s) and/or pharmacodynamical effect(s) of the sedatives on a sleeping pattern of subject 10.

One or more physiological waveforms 30 and/or one or more psychological waveforms 40 of subject 10 are monitored, particularly when subject 10 is asleep, naturally or drug-induced.

Physiological waveform(s) 30 is(are) informative of a biological functioning of subject 10, particularly when subject 10 is asleep, naturally or drug-induced. Examples of a physiological waveform 30 include, but are not limited to, an electrocardiogram (ECG) 41, a capnogram (CO₂) 42, a photoplethysmogram (PPG) 43 and an electroencephalogram (EEG) 44.

Psychological waveform(s) 40 is(are) informative of a level of conscious awareness of subject 10, particularly when subject 10 is asleep, naturally or drug-induced. Examples of a psychological waveform 40 include, but are not limited to, a chest well waveform 41, a thorax waveform 42 and an abdomen waveform 43.

For purposes of claiming and describing the present disclosure, a monitoring waveform is a physiological waveform 30 or a psychological waveform 40.

Still referring to FIG. 1, a sleep quality detection device 50 of the present disclosure categorically or quantitatively classifies a sleeping status of subject 10 as natural sleep or a drug-induced sleep based on physiological waveform(s) 30 and/or psychological waveform(s) 40 annotated with relevant pharmacological data 20 of subject 10. To this end, sleep quality detection device 50 employs a waveform pre-processor 60 and a sleep quality classifier 70, and optionally a sleep pattern analyzer 80.

For purposes of claiming and describing the present disclosure,
(1) waveform pre-processor 60 is broadly defined as any processor as known in the art of the present disclosure or hereinafter conceived that is programmed in accordance with the present disclosure for deriving a plurality of monitoring waveform segments/segment subsets from a temporal segmentation of physiological waveform(s) 30 and/or psychological waveform(s) 40 annotated with relevant pharmacological data 20 of subject 10,
(2) sleep quality classifier 70 is broadly defined any classifier including a machine learning model and/or a network of machine learning models as known in the art of the present disclosure or hereinafter conceived that is(are) trained to individually classify one or more monitoring waveform segments/segment subsets as one of a natural sleep waveform segment/segment subset or a drug-induced sleep waveform segment/segment subset, and may be further trained to individually classify each monitoring waveform segment/segment subset as a natural sleep waveform segment/segment subset, a drug-induced sleep waveform segment/segment subset or a conscious waveform segment/segment subset, and
(3) sleep pattern analyzer 80 is broadly defined as any processor as known in the art of the present disclosure or hereinafter conceived that is programmed in accordance with the present disclosure to derive one or more sleep pattern metrics from a temporal analysis of a classification by the sleep quality classifier of the monitoring waveform segment(s)/segment subset(s).

In practice, waveform pre-processor 60, sleep quality classifier 70 and sleep pattern analyzer 80 (if employed) may be wholly or partially integrated or wholly segregated within a single device platform, or may be partially integrated or wholly segregated across multiple device platforms.

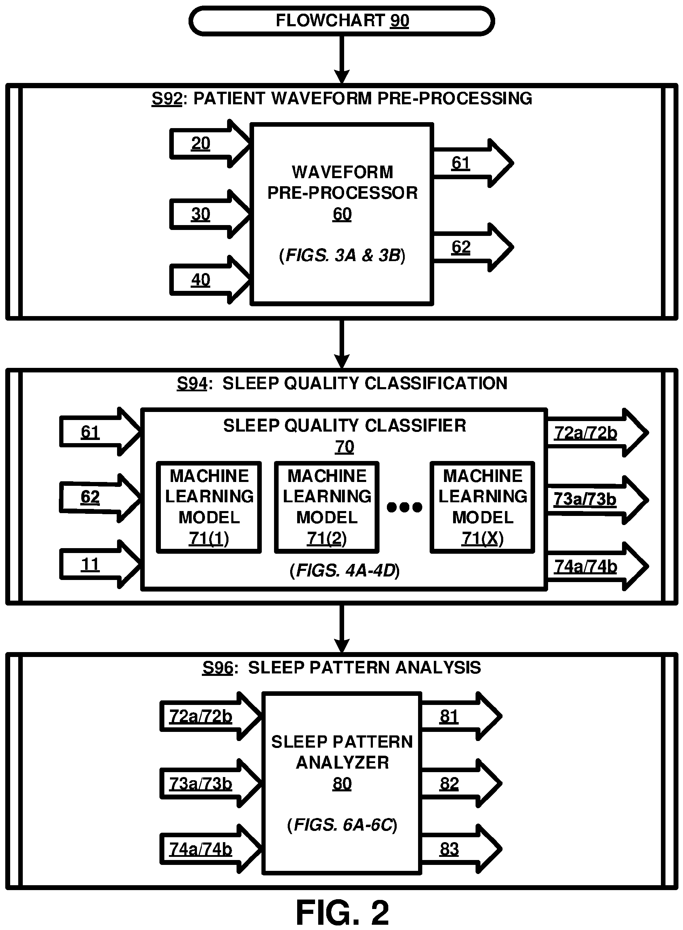

In operation, sleep quality detection device 50 executes a flowchart 90 as shown in FIG. 2 that is representative of a sleep quality detection method of the present disclosure.

Referring to FIG. 2, a stage S92 of flowchart 90 encompasses waveform pre-processor 60 deriving a plurality of monitoring waveform segments 61 from a temporal segmentation of a single monitoring waveform (i.e., a single physiological waveform 30 or a single psychological waveform 40) annotated with relevant pharmacological data 20 of subject 10, or deriving a plurality of monitoring waveform segment subsets 62 from a temporal segmentation of a plurality of monitoring waveforms (e.g., two or more of physiological waveform(s) 30 and/or psychological waveform(s) 40) annotated with relevant pharmacological data 20 of subject 10.

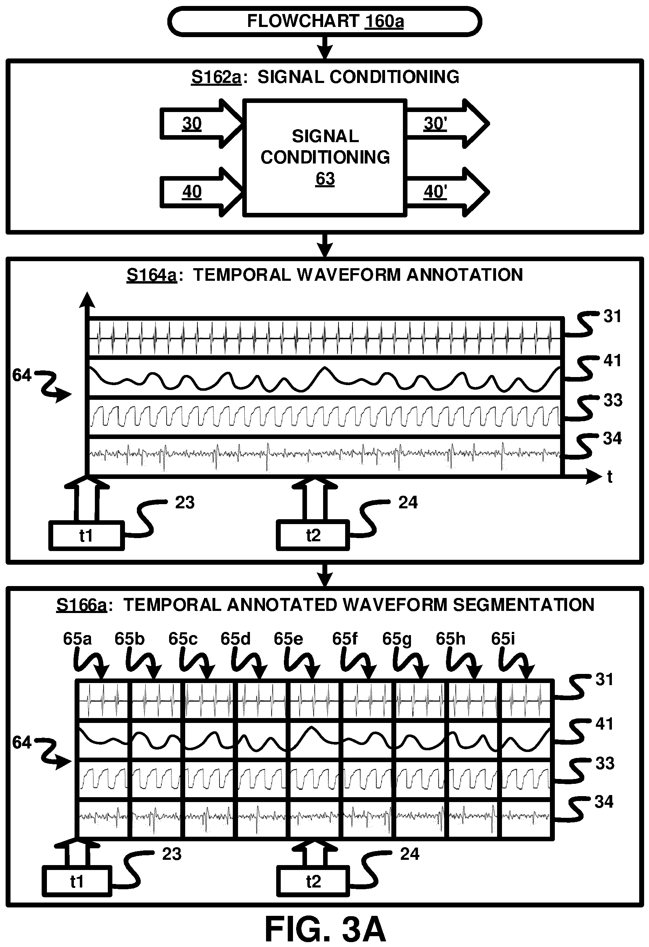

In one exemplary embodiment of stage S92, waveform pre-processor 60 executes a flowchart 160a as shown in FIG. 3A that is representative of a first embodiment of a monitoring waveform pre-processing method of the present disclosure.

In practice, the monitoring waveform(s) processed during flowchart 160a by waveform pre-processor 60 may correspond exclusively to sleeping state(s) of subject 10, or alternatively may correspond to both sleeping state(s) and a conscious state(s) of subject 10.

Referring to FIG. 3A, a stage S 162a of flowchart 160a encompasses waveform pre-processor 60 implementing a signal conditioning 63 for conditioning physiological waveform(s) 30 and/or psychological waveform(s) 40 as needed to render conditioned physiological waveform(s) 30' and/or conditioned psychological waveform(s) 40'. Examples of signal conditioning 63 include, but are not limited to, noise filtering, signal attenuation, signal amplification, analog-to-digital conversion and discretization.

In practice, signal conditioning 63 is dependent upon the output format/processing of the sources of physiological waveform(s) 30 and/or psychological waveform(s) 40 (e.g., monitors, sensors, etc.). For example, an electrocardiograph may perform noise filtering and discretization prior of an electrocardiogram (ECG) prior to outputting the ECG. As such, stage S162a may be omitted if signal conditioning is unnecessary for purposes of the present disclosure.

Still referring to FIG. 3A, a stage S164a of flowchart 160a encompasses waveform pre-processor 60 implementing a temporal waveform annotation of the unconditioned/conditioned monitoring waveform(s) of stage S162a.

In practice of a single monitoring waveform (i.e., a single physiological waveform 30 or a single psychological waveform 40), waveform pre-processor 60 annotate the single monitoring waveform for each sedative administrated during the monitoring waveform with each annotation including a name and a dose of a corresponding sedative at a time of administration of the sedative to subject 10.

For example, with a single conditioned or unconditioned electroencephalogram (EEG) 34, stage S164a shows an annotation 23 of a name and a dose of a first sedative administered to subject 10 at a time t1, and an annotation 24 of a name and a dose of a second sedative administered to subject 10 at a time t2.

In practice of a plurality of monitoring waveforms (e.g., two or more of physiological waveform(s) 30 and/or psychological waveform(s) 40), waveform pre-processor 60 temporally aligns the monitoring waveforms as a monitoring waveform set, and annotate the monitoring waveform set for each sedative administrated during the monitoring waveform set with each annotation including a name and a dose of a corresponding sedative at a time of administration of the sedative to subject 10.

For example, for an electrocardiogram (ECG) 31, a chest wall waveform 41, a capnogram (CO₂) 33 and electroencephalogram (EEG) 34, stage S164a shows a temporal alignment of the monitoring waveforms into a monitoring waveform set 64 having annotation 23 of a name and a dose of a first sedative administered to subject 10 at a time t1, and annotation 24 of a name and a dose of a second sedative administered to subject 10 at a time t2.

Still referring to FIG. 3A, a stage S166a of flowchart 160a encompasses waveform pre-processor 60 implementing a segmentation of the temporal waveform annotation of stage S164a.

In practice of a single annotated monitoring waveform (i.e., a single annotated physiological waveform 30 or a single annotated psychological waveform 40), waveform pre-processor 60 segments the single annotated monitoring waveform into equal segments of time, such as, for example, a segmentation of an annotated electroencephalogram (EEG) 34 into nine (9) segments as shown in stage S166a.

Alternatively, waveform pre-processor 60 may segment the single annotated monitoring waveform into varied segments of time, such as, for example, a segmentation of annotated electroencephalogram (EEG) 34 into equal segments of time for a time period customarily or prescribed for a sleeping state of subject 10, and a segmentation of annotated electroencephalogram (EEG) 34 into equal segments of time for a time period customarily or prescribed for a conscious state of subject 10, where a duration of the sleeping waveform segments is less than a duration of the conscious waveform segments.

In practice of a plurality of annotated monitoring waveforms (e.g., two or more of annotated physiological waveform(s) 30 and/or annotated psychological waveform(s) 40), waveform pre-processor 60 segments annotated monitoring waveform set into annotated monitoring waveform segment subsets of equal time, such as, for example, a segmentation of annotated monitoring waveform set 64 into nine (9) annotated monitoring waveform segment subsets 65a-65i of equal time as shown in stage S166a.

Alternatively, waveform pre-processor 60 may segment the annotated monitoring waveform set into annotated monitoring waveform segment subsets of varied segments of time, such as, for example, a segmentation of annotated monitoring waveform set 64 into annotated monitoring waveform segment subsets of equal time customarily or prescribed for a sleeping state of subject 10, and a segmentation of annotated monitoring waveform set 64 into annotated monitoring waveform segment subsets of equal time customarily or prescribed for a conscious state of subject 10, where a duration of the sleeping waveform segment subsets are less than a duration of the conscious waveform segments subsets.

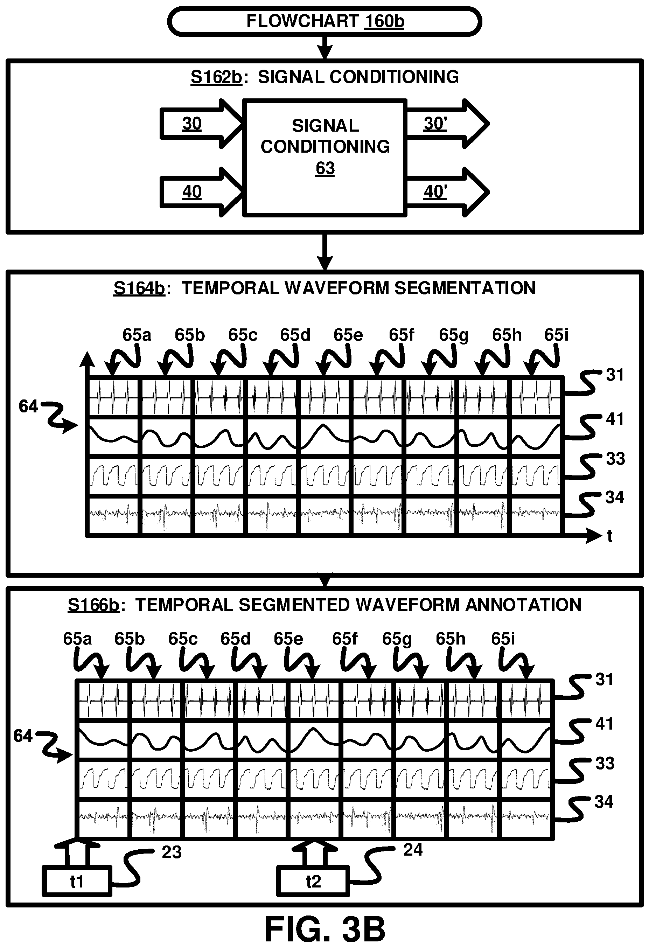

Referring back to FIG. 2, in a second exemplary embodiment of stage S92, waveform pre-processor 60 executes a flowchart 160b as shown in FIG. 3B that is representative of a second embodiment of a monitoring waveform pre-processing method of the present disclosure.

In practice, the monitoring waveform(s) processed during flowchart 160b by waveform pre-processor 60 may correspond exclusively to sleeping state(s) of subject 10, or alternatively may correspond to both sleeping state(s) and a conscious state(s) of subject 10.

Referring to FIG. 3B, a stage S162b of flowchart 160b encompasses waveform pre-processor 60 implementing a signal conditioning 63 for conditioning physiological waveform(s) 30 and/or psychological waveform(s) 40 as needed to render conditioned physiological waveform(s) 30' and/or conditioned psychological waveform(s) 40'. Examples of signal conditioning 63 include, but are not limited to, noise filtering, signal attenuation, signal amplification, analog-to-digital conversion and discretization.

In practice, signal conditioning 63 is dependent upon the output format/processing of the sources of physiological waveform(s) 30 and/or psychological waveform(s) 40 (e.g., monitors, sensors, etc.). For example, an electrocardiograph may perform noise filtering and discretization prior of an electrocardiogram (ECG) prior to outputting the ECG. As such, stage S162b may be omitted if signal conditioning is unnecessary for purposes of the present disclosure.

Still referring to FIG. 3B, a stage S166b of flowchart 160b encompasses waveform pre-processor 60 implementing a segmentation of signal pre-processing of stage S 162b.

In practice of a single monitoring waveform (i.e., a single physiological waveform 30 or a single psychological waveform 40), waveform pre-processor 60 segments the single monitoring waveform into equal segments of time, such as, for example, a segmentation of electroencephalogram (EEG) 34 into nine (9) segments as shown in stage S164b.

Alternatively, waveform pre-processor 60 may segment the single monitoring waveform into varied segments of time, such as, for example, a segmentation of electroencephalogram (EEG) 34 into equal segments of time for a time period customarily or prescribed for a sleeping state of subject 10, and a segmentation of electroencephalogram (EEG) 34 into equal segments of time for a time period customarily or prescribed for a conscious state of subject 10, where a duration of the sleeping waveform segments is less than a duration of the conscious waveform segments.

In practice of a plurality of monitoring waveforms (e.g., two or more of physiological waveform(s) 30 and/or psychological waveform(s) 40), waveform pre-processor 60 temporally aligns the monitoring waveforms into a monitoring waveform set and waveform pre-processor 60 segments the monitoring waveform set into monitoring waveform segment subsets of equal time, such as, for example, a temporal alignment of an electrocardiogram (ECG) 31, a chest wall waveform 41, a capnogram (CO₂) 33 and electroencephalogram (EEG) 34 into a monitoring waveform set 64 and a segmentation of monitoring waveform set 64 into nine (9) monitoring waveform segment subsets 65a-65i of equal time as shown in stage S166b.

Alternatively, waveform pre-processor 60 may segment the monitoring waveform set into monitoring waveform segment subsets of a varied segments of time, such as, for example, a segmentation of monitoring waveform set 64 into monitoring waveform segment subsets of equal time customarily or prescribed for a sleeping state of subject 10, and a segmentation of monitoring waveform set 64 into monitoring waveform segment subsets of equal time customarily or prescribed for a conscious state of subject 10, where a duration of the sleeping waveform segment subsets are less than a duration of the conscious waveform segment subsets.

Still referring to FIG. 3B, a stage S166b of flowchart 160b encompasses waveform pre-processor 60 implementing an annotation of the temporal waveform segmentation of stage S 164b.

In practice of a single segmented monitoring waveform (i.e., a single segmented physiological waveform 30 or a single segmented psychological waveform 40), waveform pre-processor 60 annotates the single segmented monitoring waveform for each sedative administrated during the monitoring waveform with each annotation including a name and a dose of a corresponding sedative at a time of administration of the sedative to subject 10.

For example, with a single segmented electroencephalogram (EEG) 34, stage S166b shows an annotation 23 of the first segment with a name and a dose of a first sedative administered to subject 10 at a time t1, and an annotation 24 of fifth segment with a name and a dose of a second sedative administered to subject 10 at a time t2.

In practice of a segmented monitoring waveform set, waveform pre-processor 60 annotates each monitoring waveform segment subset corresponding to a sedative administrated during the monitoring waveform set with each annotation including a name and a dose of a corresponding sedative at a time of administration of the sedative to subject 10.

For example, for a segmented monitoring waveform set 64, stage S166b shows an annotation 23 of first monitoring waveform segment subset 64a with a name and a dose of a first sedative administered to subject 10 at a time t1, and annotation 24 of a fifth monitoring waveform segment subset 64e with a name and a dose of a second sedative administered to subject 10 at a time t2.

Referring back to FIG. 2, a stage S94 of flowchart 90 involves waveform pre-processor 60 either communicating monitoring waveform segments 61 from single monitoring waveform processing to sleep quality classifier 70 or a data storage for later retrieval by sleep quality classifier 70, or alternatively communicating monitoring waveform segment subsets 62 from multiple monitoring waveform processing to sleep quality classifier 70 or a data storage for later retrieval by sleep quality classifier 70.

In one exemplary embodiment of stage S94, upon receiving a communication of monitoring waveform segments 61 or retrieving monitoring waveform segments 61 from storage, sleep quality classifier 70 implements one or more machine learning models 71 (e.g., supervised learning model(s) or semi-supervised learning model(s)) trained to individually classify one or more of the monitoring waveform segments 61 as a natural sleep waveform segment 72a or a drug-induced sleep waveform segment 73a. Machine learning model(s) 71 may also be trained to extract relevant features from monitoring waveform segments 61, or additional machine learning model(s) may be trained to extract relevant features from monitoring waveform segments 61.

Still referring to FIG. 2, in a second exemplary embodiment of stage S94, upon receiving a communication of monitoring waveform segments 61 or retrieving monitoring waveform segments 61 from storage in combination with subject demographic data 11, sleep quality classifier 70 implements one or more machine learning models 71 (e.g., supervised learning model(s) or semi-supervised learning model(s)) trained to individually classify one or more of the monitoring waveform segments 61 as a natural sleep waveform segment 72a or a drug-induced sleep waveform segment 73a based on the subject demographic data 11.

Examples of subject demographic data 11 include, but are not limited to age, height, weight, race, gender and any other characteristic that effects the metabolism of the subject.

For this embodiment, the machine learning model(s) 71 of sleep quality classifier 70 may be trained on subject demographic data 11 as input into the classification algorithm.

Alternatively, individual machine learning model(s) 71 and/or network(s) of machine learning models 71 may be trained on different aspects of subject demographic data 11. For example, sleep quality classifier 70 may include an individual machine learning model 71 or a network of machine learning models 71 trained exclusively for males, or males of a specific age range, or males of specific race, and may further include an additional individual machine learning model 71 or an additional network of machine learning models 71 trained exclusively for females, or females of a specific age range, or females of specific race.

Machine learning model(s) 71 may also be trained to extract relevant features from monitoring waveform segments 61 based on the subject demographic data 11, or additional machine learning model(s) may be trained to extract relevant features from monitoring waveform segments 61 based on the subject demographic data 11.

Still referring to FIG. 2, in a third exemplary embodiment of stage S94, upon receiving a communication of monitoring waveform segments 61 or retrieving monitoring waveform segments 61 from storage, sleep quality classifier 70 implements one or more machine learning models 71 (e.g., supervised learning model(s) or semi-supervised learning model(s)) trained to individually classify each monitoring waveform segments 61 as a natural sleep waveform segment 72a , a drug-induced sleep waveform segment 73a or a conscious state waveform segment 74a.

Again, the machine learning model(s) 71 may also be trained to extract relevant features from monitoring waveform segments 61, or additional machine learning model(s) may be trained to extract relevant features from monitoring waveform segments 61.

Still referring to FIG. 2, in a fourth exemplary embodiment of stage S94, upon receiving a communication of monitoring waveform segments 61 or retrieving monitoring waveform segments 61 from storage in combination with subject demographic data 11, sleep quality classifier 70 implements one or more machine learning models 71 (e.g., supervised learning model(s) or semi-supervised learning model(s)) trained to individually classify one or more of the monitoring waveform segments 61 as natural sleep waveform segment 72a, drug-induced sleep waveform segment 73a or conscious state waveform segment 74a based on the subject demographic data 11.

Examples of subject demographic data 11 include, but are not limited to age, height, weight, race, gender and any other characteristic that effects the metabolism of the subject.

For this embodiment, the machine learning model(s) 71 of sleep quality classifier 70 may be trained on subject demographic data 11 as input into the classification algorithm.

Alternatively, individual machine learning model(s) 71 and/or network(s) of machine learning models 71 may be trained on different aspects of subject demographic data 11. For example, sleep quality classifier 70 may include an individual machine learning model 71 or a network of machine learning models 71 trained exclusively for males, or males of a specific age range, or males of specific race, and may further include an additional individual machine learning model 71 or an additional network of machine learning models 71 trained exclusively for females, or females of a specific age range, or females of specific race.

Again, the machine learning model(s) 71 may also be trained to extract relevant features from monitoring waveform segments 61 based on the subject demographic data 11, or additional machine learning model(s) may be trained to extract relevant features from monitoring waveform segments 61 based on the subject demographic data 11.

Still referring to FIG. 2, in a fifth exemplary embodiment of stage S94, upon receiving a communication of monitoring waveform segment subsets 62 or retrieving monitoring waveform segment subsets 62 from storage, sleep quality classifier 70 implements one or more machine learning models 71 (e.g., supervised learning model(s) or semi-supervised learning model(s)) trained to individually classify one or more of the monitoring waveform segment subsets 62 as a natural sleep waveform segment subset 72a or a drug-induced sleep waveform segment subset 73a.

Machine learning model(s) 71 may also be trained to extract relevant features from monitoring waveform segment subsets 62, or additional machine learning model(s) may be trained to extract relevant features from monitoring waveform segment subsets 62.

Still referring to FIG. 2, in a sixth exemplary embodiment of stage S94, upon receiving a communication of monitoring waveform segment subsets 62 or retrieving monitoring waveform segment subsets 62 from storage in combination with subject demographic data 11, sleep quality classifier 70 implements one or more machine learning models 71 (e.g., supervised learning model(s) or semi-supervised learning model(s)) trained to individually classify one or more of the monitoring waveform segment subsets 62 as a natural sleep waveform segment subset 72a or a drug-induced sleep waveform segment subset 73a based on the subject demographic data 11.

Examples of subject demographic data 11 include, but are not limited to age, height, weight, race, gender and any other characteristic that effects the metabolism of the subject.

For this embodiment, the machine learning model(s) 71 of sleep quality classifier 70 may be trained on subject demographic data 11 as input into the classification algorithm.

Alternatively, individual machine learning model(s) 71 and/or network(s) of machine learning models 71 may be trained on different aspects of subject demographic data 11. For example, sleep quality classifier 70 may include an individual machine learning model 71 or a network of machine learning models 71 trained exclusively for males, or males of a specific age range, or males of specific race, and may further include an additional individual machine learning model 71 or an additional network of machine learning models 71 trained exclusively for females, or females of a specific age range, or females of specific race.

Machine learning model(s) 71 may also be trained to extract relevant features from monitoring waveform segment subsets 62 based on the subject demographic data 11, or additional machine learning model(s) may be trained to extract relevant features from monitoring waveform segment subsets 62 based on the subject demographic data 11.

Still referring to FIG. 2, in a seventh exemplary embodiment of stage S94, upon receiving a communication of monitoring waveform segment subsets 62 or retrieving monitoring waveform segment subsets 62 from storage, sleep quality classifier 70 implements one or more machine learning models 71 (e.g., supervised learning model(s) or semi-supervised learning model(s)) trained to individually classify each monitoring waveform segment subsets 62 as a natural sleep waveform segment subset 72a , a drug-induced sleep waveform segment subset 73a or a conscious state waveform segment subset 74a.

Again, the machine learning model(s) 71 may also be trained to extract relevant features from monitoring waveform segment subsets 62, or additional machine learning model(s) may be trained to extract relevant features from monitoring waveform segment subsets 62.

Still referring to FIG. 2, in an eighth exemplary embodiment of stage S94, upon receiving a communication of monitoring waveform segment subsets 62 or retrieving monitoring waveform segment subsets 62 from storage in combination with subject demographic data 11, sleep quality classifier 70 implements one or more machine learning models 71 (e.g., supervised learning model(s) or semi-supervised learning model(s)) trained to individually classify one or more of the monitoring waveform segment subsets 62 as natural sleep waveform segment subset 72a, drug-induced sleep waveform segment subset 73a or conscious state waveform segment subset 74a based on the subject demographic data 11.

Examples of subject demographic data 11 include, but are not limited to age, height, weight, race, gender and any other characteristic that effects the metabolism of the subject.

For this embodiment, the machine learning model(s) 71 of sleep quality classifier 70 may be trained on subject demographic data 11 as input into the classification algorithm.

Alternatively, individual machine learning model(s) 71 and/or network(s) of machine learning models 71 may be trained on different aspects of subject demographic data 11. For example, sleep quality classifier 70 may include an individual machine learning model 71 or a network of machine learning models 71 trained exclusively for males, or males of a specific age range, or males of specific race, and may further include an additional individual machine learning model 71 or an additional network of machine learning models 71 trained exclusively for females, or females of a specific age range, or females of specific race.

Again, the machine learning model(s) 71 may also be trained to extract relevant features from monitoring waveform segment subsets 62 based on the subject demographic data 11, or additional machine learning model(s) may be trained to extract relevant features from monitoring waveform segment subsets 62 based on the subject demographic data 11.

Still referring to FIG. 1, in practice, the machine learning model(s) 71 may implement one or more classification techniques as known in the art of the present disclosure or herein after conceived for categorially classifying a monitoring waveform segment 61 as a natural sleep waveform segment 72a or a drug-induced sleep waveform segment 73a (or a conscious waveform segment 74a if applicable), and for categorially classifying a monitoring waveform segment subset 62 as a natural sleep waveform segment subset 72b or a drug-induced sleep waveform segment subset 73b (or a conscious waveform segment subset 74b if applicable).

Examples of a classification technique include, but are not limited to, an artificial neural network, a deep learning neural network, a support vector machine (SVM), a decision tree, a random forest, a *k*-nearest neighbor, a Naive Bayes, a discriminant analysis and a logistic regression.

Concurrently or alternatively in practice, the machine learning model(s) 71 may implement one or more regression techniques as known in the art of the present disclosure or herein after conceived for quantifiably classifying a monitoring waveform segment 61 as a natural sleep waveform segment 72a or a drug-induced sleep waveform segment 73a (or a conscious waveform segment 74a if applicable), and for quantifiably classifying a monitoring waveform segment subset 62 as a natural sleep waveform segment subset 72b or a drug-induced sleep waveform segment subset 73b (or a conscious waveform segment subset 74b if applicable).

Examples of a regression technique include, but are not limited to, an artificial neural network, a deep learning neural network, a decision tree, a linear model, a non-linear model, a regularization, a stepwise regression and an adaptive neuro-fuzzing learning.

In practice, a recurrent neural network is a practical neural network for sleep quality classifier 70 because a recurrent neural network is configured with an internal state or memory to process a temporal sequence of input data like waveforms where the same function is performed for every element of a sequence with the output of each function being dependent on the previous computations by that function.

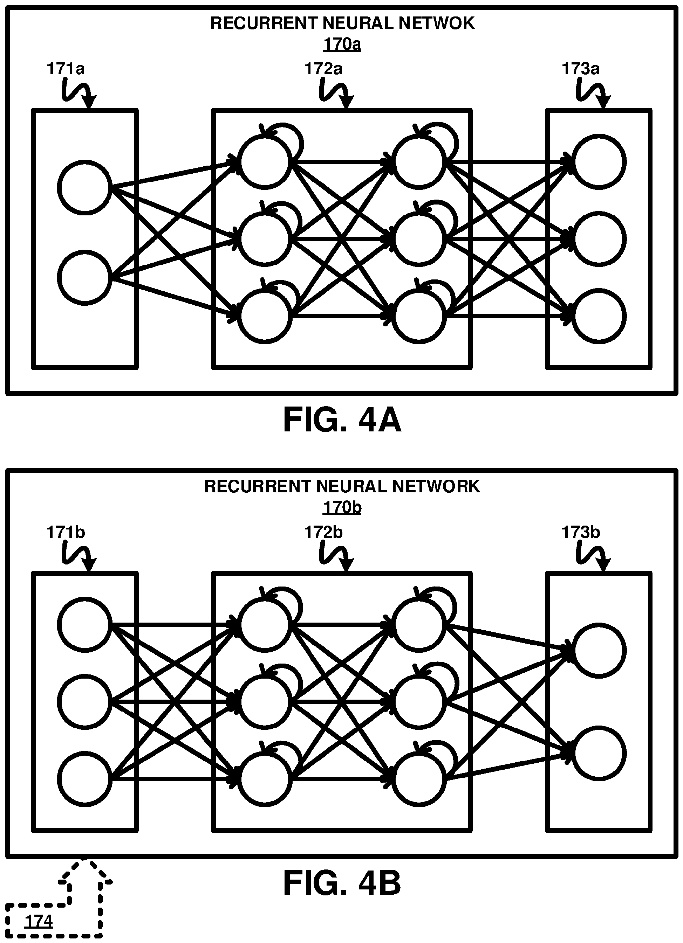

In one exemplary embodiment of sleep quality classifier 70 (FIG. 1) as shown in FIG. 4A, a recurrent neural network 170a is configured with an input layer 171a, a hidden layer 172a and an output layer 173a.

Referring to FIG. 4A, input layer 171a configured to either input a temporal sequence of monitoring waveform segments 61 (FIG. 2) and/or input a temporal sequence of monitoring waveform segment subsets 62 (FIG. 2). Input layer 171a may further configured to input subject demographic data 11 (FIG. 2) in addition to monitoring waveform segments 61 or monitoring waveform segment subsets 62.

For an input of a temporal sequence of monitoring waveform segments 61 (and subject demographic data 11 if applicable), output layer 173a is configured to output a categorical classification or a quantitative classification of each monitoring waveform segment as a natural sleep waveform segment 72a, a drug-induced sleep waveform segment 73a or a conscious waveform segment 74a.

For an input of a temporal sequence of monitoring waveform segment subsets 62 (and subject demographic data 11 if applicable), output layer 173b is configured to output a categorical classification or a quantitative classification of each monitoring waveform segment subset as a natural sleep waveform segment subset 72b, a drug-induced sleep waveform segment subset 73b or a conscious waveform segment subset 74b.

Hidden layer 172a functional maps input layer 171a to output layers 173a via layer(s) of classification neurons having trained weights and biases of an assigned activation function as known in the art of the present disclosure and hereinafter conceived Examples of an activation function includes, but are not limited to, a step function, a linear function, a sigmoid function, a tanh function and a ReLu function.

In a second exemplary embodiment of sleep quality classifier 70 (FIG. 1) as shown in FIG. 4B, a recurrent neural network 170a is configured with an input layer 171a, a hidden layer 172a and an output layer 173a.

Referring to FIG. 4B, input layer 171b configured to either input a temporal sequence of monitoring waveform segments 61 (FIG. 2) and/or input a temporal sequence of monitoring waveform segment subsets 62 (FIG. 2). Input layer 171b may further be configured to input subject demographic data 11 (FIG. 2) in addition to monitoring waveform segments 61 or monitoring waveform segment subsets 62. Input layer 171b may be configured to input sleep detection data whereby recurrent neural network 170b is only operational if the subject has been detected to be in a sleep state, or recurrent neural network 170b may be configured to input an enablement signal 174 whereby recurrent neural network 170b is only operational if enabled by the enablement signal 174.

For an input of a temporal sequence of monitoring waveform segments 61 (and subject demographic data 11 if applicable) with recurrent neural network 170b operational/enabled, output layer 173b is configured to output a categorical classification or a quantitative classification of each monitoring waveform segment as a natural sleep waveform segment 72a or a drug-induced sleep waveform segment 73a.

For an input of a temporal sequence of monitoring waveform segment subsets 62 (and subject demographic data 11 if applicable) with recurrent neural network 170b operational/enabled, output layer 173b is configured to output a categorical classification or a quantitative classification of each monitoring waveform segment subset as a natural sleep waveform segment subset 72b or a drug-induced sleep waveform segment subset 73b.

Hidden layer 172b functional maps input layer 171b to output layers 173b via layer(s) of classification neurons having trained weights and biases of an assigned activation function as known in the art of the present disclosure and hereinafter conceived. Examples of an activation function includes, but are not limited to, a step function, a linear function, a sigmoid function, a tanh function and a ReLu function.

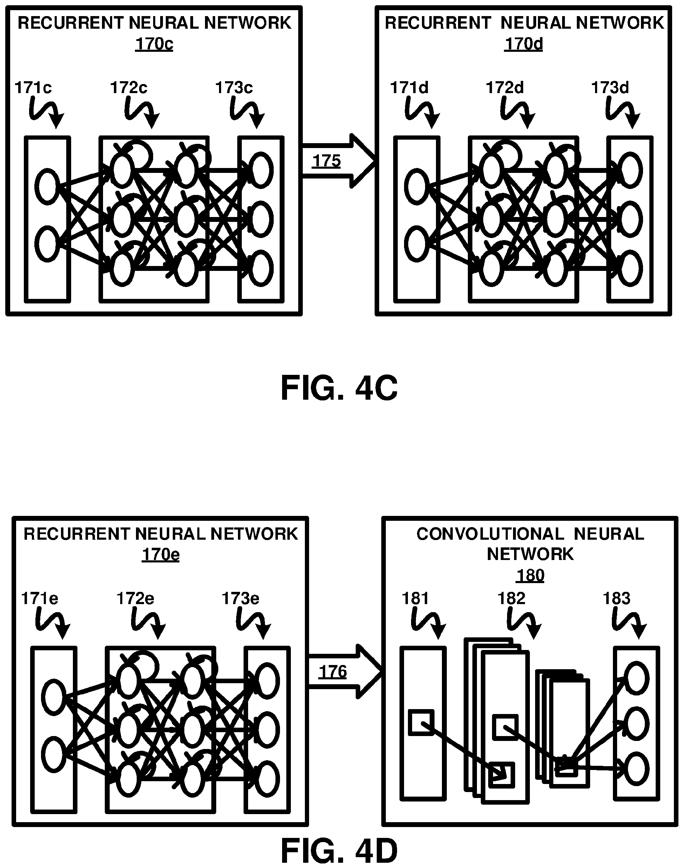

In a third exemplary embodiment of sleep quality classifier 70 as shown in FIG. 4C, a recurrent neural network (RNN) 170c feeds a classification 175 of a sleeping subject or a conscious subject to a recurrent neural network (RNN) 170d.

Referring to FIG. 4C, an input layer 171c of RNN 170c is configured to either input a temporal sequence of monitoring waveform segments 61 (FIG. 2) and/or input a temporal sequence of monitoring waveform segment subsets 62 (FIG. 2). Input layer 171b may further be configured to input subject demographic data 11 (FIG. 2) in addition to monitoring waveform segments 61 or monitoring waveform segment subsets 62.

For an input of a temporal sequence of monitoring waveform segments 61 (and subject demographic data 11 if applicable), an output layer 173c of RNN 170c is configured to output a categorical classification or a quantitative classification of each monitoring waveform segment as a sleep waveform segment or a conscious waveform segment.

For an input of a temporal sequence of monitoring waveform segment subsets 62 (and subject demographic data 11 if applicable), output layer 173c of RNN 170c is configured to output a categorical classification or a quantitative classification of each monitoring waveform segment subset as sleep waveform segment subset or a conscious waveform segment subset.

A hidden layer 172c of RNN 170c functional maps input layer 171c to output layers 173c via layer(s) of classification neurons having trained weights and biases of an assigned activation function as known in the art of the present disclosure and hereinafter conceived. Examples of an activation function includes, but are not limited to, a step function, a linear function, a sigmoid function, a tanh function and a ReLu function.

In addition to inputting classification 175, input layer 171d of RNN 170d is configured to either input a temporal sequence of monitoring waveform segments 61 (FIG. 2) and/or input a temporal sequence of monitoring waveform segment subsets 62 (FIG. 2). Input layer 171d may further be configured to input subject demographic data 11 (FIG. 2) in addition to classification 175, and monitoring waveform segments 61 or monitoring waveform segment subsets 62.

For an input of classification 175 and a temporal sequence of monitoring waveform segments 61 (and subject demographic data 11 if applicable), an output layer 173d of RNN 170d is configured to output a categorical classification or a quantitative classification of each monitoring waveform segment as a natural sleep waveform segment or a drug-induced sleep waveform segment.

For an input of classification 175 and a temporal sequence of monitoring waveform segment subsets 62 (and subject demographic data 11 if applicable), output layer 173d of RNN 170d is configured to output a categorical classification or a quantitative classification of each monitoring waveform segment subset as a natural sleep waveform segment subset or a drug-induced waveform segment subset.

A hidden layer 172d of RNN 170d functional maps input layer 171d to output layers 173d via layer(s) of classification neurons having trained weights and biases of an assigned activation function as known in the art of the present disclosure and hereinafter conceived. Examples of an activation function includes, but are not limited to, a step function, a linear function, a sigmoid function, a tanh function and a ReLu function.

In a fourth exemplary embodiment of sleep quality classifier 70 as shown in FIG. 4D, a recurrent neural network 170e feeds a classification 176 of a natural sleeping subject, a drug-induced sleeping subject or (a conscious subject if applicable) to a convolutional neural network 180.

In practice, recurrent neural network 170e may be configured with an input layer 171e, a hidden layer 172e and an output layer 173e similar to recurrent neural network 170a (FIG. 4A) as previously described herein, similar to recurrent neural network 170b (FIG. 4B) as previously describe herein, or similar to recurrent neural network 170d (FIG. 4C) as previously describe herein.

In addition to inputting classification 176, an input layer 180 of CNN 180 is configured to either input a temporal sequence of monitoring waveform segments 61 (FIG. 2) and/or input a temporal sequence of monitoring waveform segment subsets 62 (FIG. 2). Input layer 180 may further be configured to input subject demographic data 11 (FIG. 2) in addition to classification 176, and monitoring waveform segments 61 or monitoring waveform segment subsets 62.

For an input of classification 176 and a temporal sequence of monitoring waveform segments 61 (and subject demographic data 11 if applicable), a fully connected layer 183 of CNN 180 is configured to output extracted features from monitoring waveform segments 61 indicative of the classification of each monitoring waveform segment 61.

For example, fully connected layer 183 of CNN 180 will output extracted features of a physiological waveform (e.g., an electrocardiogram) or a psychological waveform (e.g., a chest cavity waveform) corresponding to a classified natural sleep waveform segment, a classified drug-induced sleep waveform segment or if applicable, a classified conscious waveform segment.

For an input of classification 176 and a temporal sequence of monitoring waveform segment subsets 62 (and subject demographic data 11 if applicable), fully connected layer 183 of CNN 180 is configured to output extracted features from monitoring waveform segment subsets 62 indicative of the classification of each monitoring waveform segment subsets 62.

For example, fully connected layer 183 of CNN 180 will output extracted features of physiological waveform(s) (e.g., an electrocardiogram and/or an electroencephalogram) and/or psychological waveform (e.g., a chest cavity waveform and/or an abdomen waveform) corresponding to a classified natural sleep waveform segment subset, a classified drug-induced sleep waveform segment subset, or if applicable, a classified conscious waveform segment subset.

Convolutional/pooling layers 182 of CNN 180 functional map input layer 181 to fully connected layer 183 via convolution filters and feature maps as known in the art of the present disclosure and hereinafter conceived for the feature extraction.

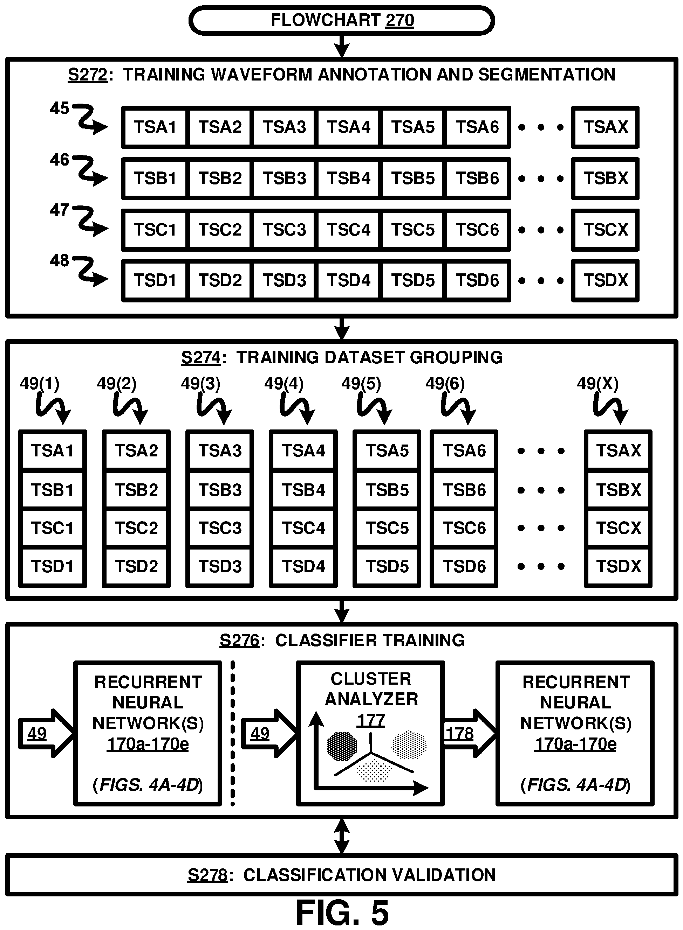

FIG. 5 illustrates a flowchart 270 representative of a sleep quality classifier training method in accordance with the present disclosure for training train sleep quality classifiers 70 (FIG. 1) of the present disclosure, particularly the recurrent neural networks of of FIGS. 4A-4D.

Referring to FIG. 5, a stage S272 of flowchart 270 encompasses annotation and segmentation of training datasets of physiological waveform(s) (e.g., an electrocardiogram and/or an electroencephalogram) and/or psychological waveform (e.g., a chest cavity waveform and/or an abdomen waveform) in accordance with the present disclosure.

For example, as shown in stage S272, (1) a training dataset 45 may consist of an X number of annotated electrocardiogram segments for a subject A, (2) a training dataset 46 may consist of an X number of annotated electrocardiogram segments for a subject B, (3) a training dataset 47 may consist of an X number of annotated electrocardiogram segments for a subject C, and (4) a training dataset 48 may consist of an X number of annotated electrocardiogram segments for a subject D.

By further example, (1) training dataset 45 may consist of an X number of annotated electrocardiogram, electroencephalogram and chest cavity segment subsets for subject A, (2) training dataset 46 may consist of an X number of annotated electrocardiogram, electroencephalogram and chest cavity segment subsets for subject B, (3) training dataset 47 may consist of an X number of annotated electrocardiogram, electroencephalogram and chest cavity segment subsets for subject C, and (4) training dataset 48 may consist of an X number of annotated electrocardiogram, electroencephalogram and chest cavity segment subsets for subject D.

In practice, flowchart 160a (FIG. 3A) or flowchart 160b (FIG. 3B) may be implemented during stage S272.

Also in practice, the time elapsed between medication administration and the start of each segment/segment subset will be calculated to facilitate modelling the effect of medications over time. Specifically, as the medication is metabolized, its effectiveness changes over time, based on its pharmacokinetic and pharmacodynamics properties. Therefore, time segments/segment subsets recorded at different time intervals will represent different degree of sedative effect.

Further in practice, each training dataset may include subject demographics such as age, weight, race, gender, which effects the metabolism.

Still referring to FIG. 5, a stage S274 of flowchart 270 encompasses a grouping together of segments/segment subsets from different subjects (or the same subject) with same or substantially the same time difference between medication administrations of the same or similar sedative will enable the sleep quality classifier to learn features characteristic of that segment/segment subset.

For example, as shown in stage S274, each grouping 49(1) to 49(X) of segments/segment subsets from different subjects A-D with same or substantially the same time difference between medication administrations of the same or similar sedative will enable the sleep quality classifier to learn features characteristic of that segment/segment subset.

Still referring to FIG. 5, a stage S276 of flowchart 270 encompasses a training of a sleep quality classifier 70 (FIG. 1) of the present disclosure based on the grouped segments/segment subset(s) of stage S274 to thereby identify waveform patterns associated with sleep and wakefulness and classify the sleep phases into drug induced vs natural sleep.

In one exemplary embodiment of stage S276, a single recurrent neural network may be trained based on the grouped segments/segment subset(s) of stage S274 via back propagation or other training techniques as known in the art of the present disclosure or herein after conceived to thereby identify waveform patterns associated with sleep and consciousness and classify the sleep phases into drug induced vs natural sleep (e.g., recurrent neural network 170a of FIG. 4A).

In a second exemplary embodiment of stage S276, a single recurrent neural network may be trained based on the grouped segments/segment subset(s) of stage S274 via back propagation or other training techniques as known in the art of the present disclosure or herein after conceived to thereby identify waveform patterns associated with sleep only and classify the sleep phases into drug induced vs natural sleep (e.g., recurrent neural network 170b of FIG. 4B).

In a third exemplary embodiment of stage S276, a first recurrent neural network may be trained based on the grouped segments/segment subsets of stage S274 via back propagation or other training techniques as known in the art of the present disclosure or herein after conceived to thereby identify waveform patterns associated with sleep and consciousness and (e.g., recurrent neural network 170c of FIG. 4C), and a second recurrent neural network may be trained based on the grouped segments/segment subset(s) previously identified as sleep waveform groups via back propagation or other training techniques as known in the art of the present disclosure or herein after conceived to thereby identify waveform patterns associated with sleep only and classify the sleep phases into drug induced vs natural sleep (e.g., recurrent neural network 170d of FIG. 4C).

In a fourth exemplary embodiment of stage S276, prior to training a recurrent neural network, a cluster analyzer 177 may be utilized to properly label each grouped segments/segment subsets of stage S274 as natural sleep subjects, drug-induced sleep subjects or if applicable, conscious subjects.

In practice, cluster analyzer 177 is an unsupervised machine learning model implementing a clustering techniques as known in the art of the present disclosure or hereinafter conceived for finding hidden patterns and intrinsic structures in the temporal sequence of grouped segments/segment subsets. Examples of a clustering technique include, but are not limited to, *k*-means clustering, *k*-medoids clustering, hierarchical clustering, Gaussian mixture models, hidden Markov models, self-organizing maps, fuzzy c-means clustering, and subtractive clustering.

Still referring to FIG. 5, a stage S278 of flowchart 270 encompasses a validated on the training data set with expert (clinician) annotation to thereby capture additional features from clinical knowledge and incorporate sleep quality classifier.

In practice, stages S272-S278 may be repeated as required or preferred to obtain a fully and accurate trained sleep quality classifier.

Referring back to FIG. 2, a stage S96 of flowchart 90 involves sleep quality classifier 70 communicating classified natural sleep waveform segment(s)/segment subset(s)72a/72b, classified drug-induced waveform segment(s)/segment subset(s) 73a/73b and if applicable, classified conscious waveform segment(s)/segment subset(s) 74a/74b to sleep pattern analyzer 80, which in derives one or more sleep pattern metrics from a temporal analysis of the classifications.

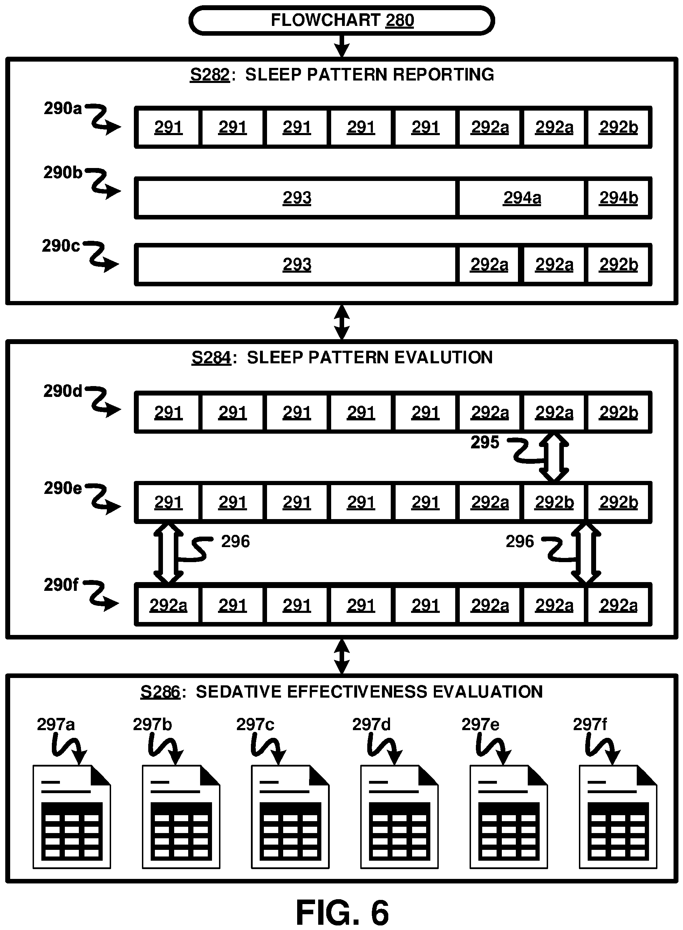

In one exemplary embodiment of stage S96, sleep pattern analyzer 80 implements a flowchart 280 as shown in FIG. 6 that is representative of a sleep pattern analysis method in accordance with the present disclosure.

Referring to FIG. 6, a stage S282 of flowchart 280 encompasses sleep pattern analyzer 80 generating a daily/weekly/monthly report(s) outlining a duration of time spent sleeping and analysis of sleep cycles, and more particularly reporting information on a proportion of drug induced sleep and natural sleep.

In practice, the reporting may be in the form of rolling time window(s) (i.e. continuously) and/or a discrete time window(s) (i.e. for each segment).

For example, as shown in stage S282, a sedative may be given to a subject every night at 9pm and sleep pattern analyzer 80 generates a daily report 290a starting at 6am of eight (8) three (3) hour segments of discretely delineating five (5) conscious windows 291 from 6am to 9pm, two (2) drug-induced sleep windows 292a from 9pm to 3am, and one (1) natural sleep window 292b from 3am to 6am.

By further example, as shown in stage S282, a sedative may be given to a subject every night at 9pm and sleep pattern analyzer 80 generates a daily report 290b starting at 6am of eight (8) three (3) hour segments of continuously delineating conscious window 293 from 6am to 9pm, drug-induced sleep window 294a from 9pm to 3am, and natural sleep window 294b from 3am to 6am.

By additional example, as shown in stage S284, a daily report 290c generated by sleep pattern analyzer 80 may have a combination of continuous conscious window 293 and discrete sleep windows 292a and 292b.

Further in practice, a notification may be sent to relevant personnel (e.g., care provider) and/or relevant systems (e.g., electronic record systems) to indicate when a subject is sleeping and shouldn't be disturbed unless necessary.

Still referring to FIG. 6, a stage S284 of flowchart 280 encompasses sleep pattern analyzer 80 evaluating sleep patterns from the daily/weekly/monthly reporting of stage S282.

In one exemplary embodiment of stage S284, sleep pattern analyzer 80 compares a current report to one or more previous reports and computes and identifies trends in sleep patterns including, but not limited to, a duration of sleep, sleep stage cycling, periods of continuous sleep and a proportion of drug induced vs. natural sleep duration correlated with the drug dose. The trends in sleep patterns may be sent to relevant personnel (e.g., care provider) and/or relevant systems (e.g., electronic record systems), particularly when positive/negative changes to sleep patterns are identified (e.g., a sleep duration or duration of continuous sleep changes abruptly).

For example, as shown in stage S284, sleep pattern analyzer 80 may identify a first sleep pattern change from a reporting 290d of two (2) drug-induced sleep windows 292a from 9pm to 3am and one (1) natural sleep window 292b from 3am to 6am to a reporting 290e of one (1) drug-induced sleep window 292a from 9pm to 12am and two (2) natural sleep windows 292b from 12am to 6am.

By further example, as shown in stage S284, sleep pattern analyzer 80 may identify a second sleep pattern change from a reporting 290e of one (1) drug-induced sleep window 292a from 9pm to 12am and two (2) (natural sleep windows 292b from 12am to 6am to a reporting 290f of four (4) drug-induced sleep windows 292a from 9pm to 9am.

Still referring to FIG. 6, a stage S286 of flowchart 280 encompasses sleep pattern analyzer 80 evaluating a sedative effectiveness from the sleep pattern evaluations of state S284.

In one exemplary embodiment of stage S286, sleep pattern analyzer 80 sleep compares the duration of sleep (total and longest continuous period) and the duration of drug- induced sleep over days in the same subject as correlated to each dose of sedative(s) to thereby facilitate an evaluation of sedative effectiveness in a subject and any changes in sedative effectiveness. Sleep pattern analyzer 80 notifies relevant personnel (e.g., care provider) and/or relevant systems (e.g., electronic record systems) if the sedative is deemed to no longer be effective for the subject as previously so the dose may be changed or the sedative can be changed.

For example, as shown in stage S286, sleep pattern analyzer 80 may evaluate a series of sleep pattern evaluations 297a-297f to ascertain a sedative effectiveness in a subject and any changes in sedative effectiveness.

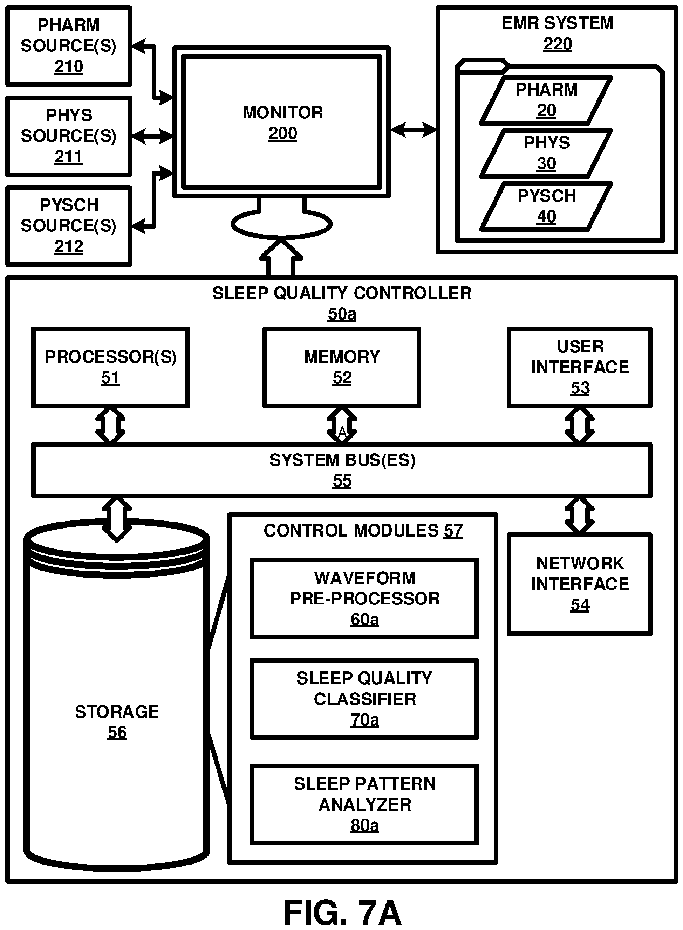

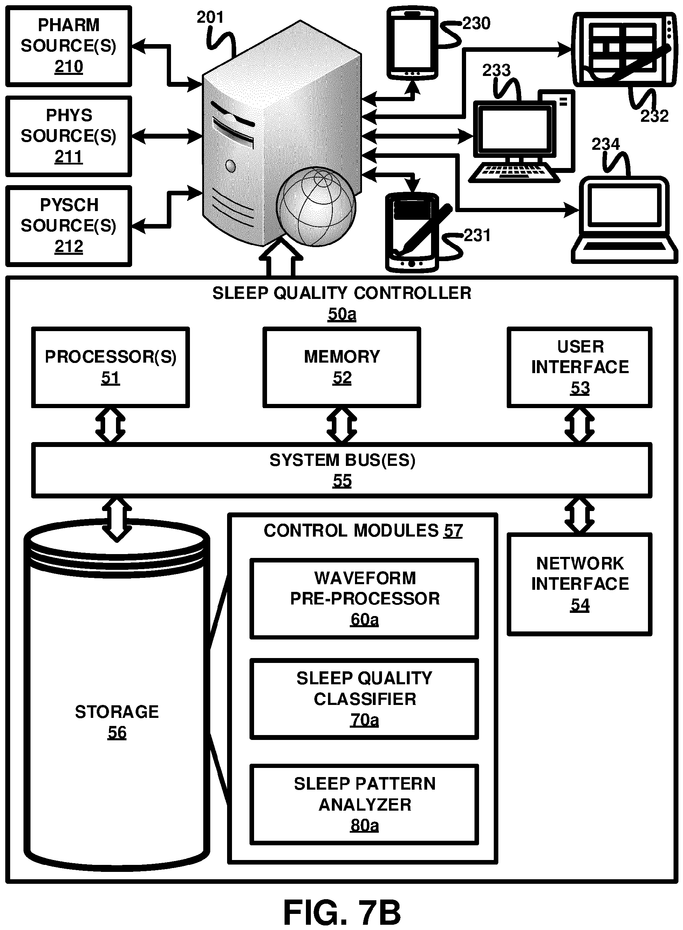

To further facilitate an understanding of the present disclosure, the following description of FIGS. 7A and 7B teaches exemplary embodiments of sleep quality detection controllers in accordance with the present disclosure. From the description of FIGS. 7A and 7B, those having ordinary skill in the art of the present disclosure will appreciate how to apply the present disclosure to make and use additional embodiments of sleep quality detection controllers in accordance with the present disclosure.

Referring to FIGS. 7A and 7B, an exemplary embodiment of sleep quality device 50 (FIG. 1) is a sleep quality controller 50a employing one or more processor(s) 51, memory 52, a user interface 53, a network interface 54, and a storage 56 interconnected via one or more system buses 55.

Each processor 51 may be any hardware device, as known in the art of the present disclosure or hereinafter conceived, capable of executing instructions stored in memory 52 or storage or otherwise processing data. In a non-limiting example, the processor(s) 51 may include a microprocessor, field programmable gate array (FPGA), application-specific integrated circuit (ASIC), or other similar devices.

The memory 52 may include various memories, as known in the art of the present disclosure or hereinafter conceived, including, but not limited to, L1, L2, or L3 cache or system memory. In a non-limiting example, the memory 52 may include static random access memory (SRAM), dynamic RAM (DRAM), flash memory, read only memory (ROM), or other similar memory devices.

The user interface 53 may include one or more devices, as known in the art of the present disclosure or hereinafter conceived, for enabling communication with a user such as an administrator. In a non-limiting example, the user interface may include a command line interface or graphical user interface that may be presented to a remote terminal via the network interface 54.

The network interface 54 may include one or more devices, as known in the art of the present disclosure or hereinafter conceived, for enabling communication with ventilation system 220, pharmacological source(s) 250, physiological source(s) 260, psychological source(s) 270 and an electronic medical records system 210. In a non-limiting example, the network interface 54 may include a network interface card (NIC) configured to communicate according to the Ethernet protocol. Additionally, the network interface 54 may implement a TCP/IP stack for communication according to the TCP/IP protocols. Various alternative or additional hardware or configurations for the network interface 54 will be apparent.

The storage 56 may include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 56 may store instructions for execution by the processor(s) 51 or data upon with the processor(s) 51 may operate. For example, the storage 56 may store a base operating system for controlling various basic operations of the hardware.

The storage 56 also stores application modules in the form of executable software/firmware for implementing the various functions of a sleep quality device 50 as previously described in the present disclosure including, but not limited to, a waveform pre-processor 60a, a sleep quality classifier 70a and a sleep pattern analyzer 80a.

As shown in FIG. 7A, sleep quality controller 50a may be incorporated within or linked to a monitor 200, such as, for example, a sleep monitor, a physiological monitor (e.g., an electrocardiograph) or a psychological monitor (e.g., a ventilation monitor).

In practice, monitor 200 may (1) generate pharmacological data 20 (FIG. 1) from user input and/or receive pharmacological data 20 from one or more pharmacological sources 210 (e.g., a local database), (2) generate physiological data 30 (FIG. 1) from internal applications (e.g., an electrocardiogram application) receiving physiological signals from one or more physiological sources 211 (e.g., ECG lead system) and/or physiological data 30 from one or more physiological sources 211 (e.g., an electrocardiograph), and (3) generate psychological data 40 (FIG. 1) from internal applications (e.g., a ventilation application) receiving a psychological signals from one or more psychological sources 212 (e.g., a chest sensor) and/or a psychological data 40 from one or more psychological sources 212 (e.g., a ventilator).

Concurrently or alternatively, monitor 200 may receive pharmacological data 20, physiological data 30 and/or psychological data 40 from a database system, such as, for example, an electronic medical records system 220 as shown.

Also in practice, waveform pre-processor 60a may alternatively be incorporated within pharmacological source(s) 210, physiological source(s) 211, psychological source(s) 212 and/or database system(s) (e.g., EMR system 220) whereby monitoring waveform segments/segment subsets generated by waveform pre-processor 60a are communicated to monitor 200.

Further in practice, sleep pattern analyzer 80a may be alternatively be incorporated within pharmacological source(s) 210, physiological source(s) 211, psychological source(s) 212 and/or database system(s) (e.g., EMR system 220) whereby classifications of natural sleep, drug-induced sleep and consciousness (if applicable) may be communicated by sleep quality classifier 70a to sleep pattern analyzer 80a for the derivation of sleep pattern metrics.

As shown in FIG. 7B, sleep quality controller 50a may be incorporated within or linked to a server 201, such as, for example, a server of an electronic medical records system 200 (FIG. 7A).

In practice, server 201 may (1) generate pharmacological data 20 (FIG. 1) from user input and/or receive pharmacological data 20 from one or more pharmacological sources 210 (e.g., a local database), (2) generate physiological data 30 (FIG. 1) from internal applications (e.g., an electrocardiogram application) receiving physiological signals from one or more physiological sources 211 (e.g., ECG lead system) and/or physiological data 30 from one or more physiological sources 211 (e.g., an electrocardiograph), and (3) generate psychological data 40 (FIG. 1) from internal applications (e.g., a ventilation application) receiving a psychological signals from one or more psychological sources 212 (e.g., a chest sensor) and/or a psychological data 40 from one or more psychological sources 212 (e.g., a ventilator).

Various clinician devices will able to access server 201 for classifications of natural sleep, drug-induced sleep and consciousness by sleep quality classifier 70a, and/or derivations of sleep pattern metric(s) by sleep pattern analyzer 80a. Examples of such devices include, but are not limited to, a mobile phone 230, a smart device 231, a mobile pad 232 and a workstation in the form of a desktop 233 or a laptop 234.

Also in practice, waveform pre-processor 60a may alternatively be incorporated within pharmacological source(s) 210, physiological source(s) 211, psychological source(s) 212 and/or database system(s) (e.g., EMR system 220) whereby monitoring waveform segments/segment subsets generated by waveform pre-processor 60a are communicated to server 201.

Further in practice, sleep pattern analyzer 80a may be alternatively be incorporated within clinician devices (e.g., devices 230-234) whereby classifications of natural sleep, drug-induced sleep and consciousness (if applicable) may be communicated by sleep quality classifier 70a to sleep pattern analyzer 80a for the derivation of sleep pattern metrics.

Referring to FIGS. 1-7, those having ordinary skill in the art of the present disclosure will appreciate numerous benefits of the present disclosure including, but not limited to, an intelligent discrimination between natural sleep and drug-induced sleep by a subject that facilitate an evaluation of a sleep quality by the subject that provide critical information on a well-being of the subject.

Further, as one having ordinary skill in the art will appreciate in view of the teachings provided herein, structures, elements, components, etc. described in the present disclosure/specification and/or depicted in the Figures may be implemented in various combinations of hardware and software, and provide functions which may be combined in a single element or multiple elements. For example, the functions of the various structures, elements, components, etc. shown/illustrated/depicted in the Figures can be provided through the use of dedicated hardware as well as hardware capable of executing software in association with appropriate software for added functionality. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar function, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the various and numerous inventions of the present disclosure (which embodiments are intended to be illustrative and not limiting), it is noted that modifications and variations can be made by persons skilled in the art in light of the teachings provided herein, including the Figures. It is therefore to be understood that changes can be made in/to the preferred and exemplary embodiments of the present disclosure which are within the scope of the embodiments disclosed herein.

Moreover, it is contemplated that corresponding and/or related systems incorporating and/or implementing the device/system or such as may be used/implemented in/with a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A sleep quality detection device (50), comprising:
a waveform pre-processor (60) configured to derive a plurality of monitoring waveform segments from a temporal segmentation of at least one monitoring waveform temporally annotated with pharmacological data; and
a sleep quality classifier (70) including at least one machine learning model trained to individually classify at least one monitoring waveform segment as one of a natural sleep waveform segment and a drug-induced sleep waveform segment.

2. The sleep quality detection device (50) of claim 1,
wherein the waveform pre-processor (60) configured to derive the plurality of monitoring waveform segments from the temporal segmentation of at least one monitoring waveform temporally annotated with pharmacological data includes:
the waveform pre-processor (60) configured to derive a plurality of monitoring waveform segment subsets from a temporal segmentation of a plurality of monitoring waveforms annotated with pharmacological data; and
wherein the sleep quality classifier (70) including the at least one machine learning model trained to individually classify the at least one of the monitoring waveform segment as one of the natural sleep waveform segment and the drug-induced sleep waveform segment includes:
the at least one machine learning model trained to individually classify at least one of the monitoring waveform segment subset as one of a natural sleep waveform segment subset and a drug-induced sleep waveform segment subset.

3. The sleep quality detection device (50) of claim 1, wherein the at least one machine learning model is trained to individually classify each monitoring waveform segment as one of the natural sleep waveform segment, the drug induced sleep waveform segment and a conscious waveform segment.

4. The sleep quality detection device (50) of claim 3,
wherein the waveform pre-processor (60) configured to derive the plurality of monitoring waveform segments from the temporal segmentation of at least one monitoring waveform temporally annotated with pharmacological data includes:
the waveform pre-processor (60) configured to derive a plurality of monitoring waveform segment subsets from a temporal segmentation of a plurality of monitoring waveforms annotated with pharmacological data; and
wherein the sleep quality classifier (70) including the at least one machine learning model trained to individually classify each monitoring waveform segment as one of the natural sleep waveform segment, the drug-induced sleep waveform segment and the conscious waveform segment includes:
the at least one machine learning model trained to individually classify each monitoring waveform segment subset as one of a natural sleep waveform segment subset, a drug-induced sleep waveform segment subset and a conscious waveform segment subset.

5. The sleep quality detection device (50) of claim 2 or 4, wherein the waveform pre-processor (60) configured to derive the plurality of monitoring waveform segment subsets from the temporal segmentation of a plurality of monitoring waveforms annotated with pharmacological data includes:
the waveform pre-processor (60) configured to temporally align the plurality of monitoring waveforms into a monitoring waveform set; and
the waveform pre-processor (60) further configured to one of:
annotate the monitoring waveform set with the pharmacological data and segment the annotated monitoring waveform set into a plurality of monitoring waveform segment subsets; and
segment the monitoring waveform set into a plurality of monitoring waveform segment subsets and annotate at least one of the monitoring waveform segment subsets with pharmacological data.

6. The sleep quality detection device (50) of claim 1, further comprising:
a sleep pattern analyzer (80) configured to derive at least one sleep pattern metric from a temporal analysis of a classification by the sleep quality classifier (70) of the at least one monitoring waveform segment as one of the natural sleep waveform segment and the drug-induced sleep waveform segment.

7. The sleep quality detection device (50) of claim 3, further comprising:
a sleep pattern analyzer (80) configured to derive at least one sleep pattern metric from a temporal analysis of a classification by the sleep quality classifier (70) of the each monitoring waveform segment subset as one of the natural sleep waveform segment subset, the drug-induced sleep waveform segment subset and the conscious waveform segment subset.

8. A sleep quality detection method, comprising:
deriving a plurality of monitoring waveform segments from a temporal segmentation of at least one monitoring waveform temporally annotated with pharmacological data; and
individually classifying, via at least one machine learning model, at least one monitoring waveform segment as one of a natural sleep waveform segment and a drug-induced sleep waveform segment.

9. The sleep quality detection method of claim 8, further comprising:
deriving at least one sleep pattern metric from a temporal analysis of a classification of the at least one monitoring waveform segment as one of the natural sleep waveform segment and the drug-induced sleep waveform segment.

10. The sleep quality detection method of claim 8,
wherein the deriving the plurality of monitoring waveform segments from the temporal segmentation of at least one monitoring waveform temporally annotated with pharmacological data includes:
deriving a plurality of monitoring waveform segment subsets from a temporal segmentation of a plurality of monitoring waveforms annotated with pharmacological data; and
wherein the individually classifying, via the at least one machine learning model, the at least one of the monitoring waveform segment as one of the natural sleep waveform segment and the drug-induced sleep waveform segment includes:
individually classifying, via the at least one machine learning model, at least one of the monitoring waveform segment subset as one of the natural sleep waveform segment subset and a drug-induced sleep waveform segment subset.

11. The sleep quality detection method of claim 8, wherein the individually classifying, via the at least one machine learning model, the at least one of the monitoring waveform segment as one of the natural sleep waveform segment and the drug-induced sleep waveform segment includes:
individually classifying, via the at least one machine learning model, each monitoring waveform segment as one of the natural sleep waveform segment, the drug induced sleep waveform segment and a conscious waveform segment.

12. The sleep quality detection method of claim 11,
wherein the deriving the plurality of monitoring waveform segments from the temporal segmentation of at least one monitoring waveform temporally annotated with pharmacological data includes:
deriving a plurality of monitoring waveform segment subsets from a temporal segmentation of a plurality of monitoring waveforms annotated with pharmacological data; and
wherein the individually classifying, via the at least one machine learning model, the at least one of the monitoring waveform segment as one of the natural sleep waveform segment and the drug-induced sleep waveform segment includes:
individually classifying, via the at least one machine learning model, each monitoring waveform segment subset as one of a natural sleep waveform segment subset, a drug-induced sleep waveform segment subset and a conscious waveform segment subset.

13. The sleep quality detection method of claim 10 or 12, wherein the deriving the plurality of monitoring waveform segment subsets from the temporal segmentation of a plurality of monitoring waveforms annotated with pharmacological data includes:
temporally aligning the plurality of monitoring waveforms; and
one of:
annotating the temporal alignment of the plurality of monitoring waveforms with the pharmacological data and segment the annotated temporal alignment of the plurality of monitoring waveforms into the monitoring waveform segment subsets; and
segmenting the temporal alignment of the plurality of monitoring waveforms into the monitoring waveform segment subsets and annotate at least one of the monitoring waveform segments with pharmacological data.

14. A sleep quality detection controller (50a), comprising:
at least one processor (51), and
a non-transitory machine-readable storage medium (52, 56) encoded with instructions executable by the at least one processor (51) to carry out the method of any of the preceding method claims 8-13.

15. A computer program product comprising instructions executable by at least one processor (51) to carry out the method of any of the preceding method claims 8-13.
